(19) Europäisches Patentamt
European Patent Office
Office européen des brevets

(11) EP 0 713 381 B1

(12) EUROPEAN PATENT SPECIFICATION

(45) Date of publication and mention of the grant of the patent: **21.01.1998 Bulletin 1998/04**

(21) Application number: **94924867.8**

(22) Date of filing: **10.08.1994**

(51) Int. Cl.$^6$: **A61K 7/02**, A61K 7/50

(86) International application number: **PCT/EP94/02670**

(87) International publication number: **WO 95/05145 (23.02.1995 Gazette 1995/09)**

(54) **CLEANSING COMPOSITION CONTAINING HYDROXY ALKANOATE DERIVATIVES**

REINIGUNGSZUSAMMENSETZUNGEN DIE HYDROXYALKANOATDERIVATE ENTHALTEN

COMPOSITION DEMAQUILLANTE CONTENANT DES DERIVES D'HYDROXYALCANOATE

(84) Designated Contracting States:
**CH DE ES FR GB IT LI NL SE**

(30) Priority: **13.08.1993 GB 9316984**

(43) Date of publication of application:
**29.05.1996 Bulletin 1996/22**

(73) Proprietors:
- **UNILEVER PLC**
  **London EC4P 4BQ (GB)**
  Designated Contracting States:
  **GB**
- **UNILEVER N.V.**
  **3013 AL Rotterdam (NL)**
  Designated Contracting States:
  **CH DE ES FR IT LI NL SE**

(72) Inventors:
- **LYLE, Ian Gardner,**
  **60 Highland Avenue**
  **Aston Park**
  **Clwyd CH5 1XQ (GB)**
- **ROSSER, David Arthur,**
  **The Sycamores**
  **The Mount**
  **Merseyside L60 4RD (GB)**

(74) Representative:
**Elliott, Peter William et al**
**Unilever plc**
**Patent Division**
**Colworth House**
**Sharnbrook**
**Bedford MK44 1LQ (GB)**

(56) References cited:
**EP-A- 0 150 914** **EP-A- 0 541 347**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 713 381 B1

## Description

This invention relates to cleansing compositions. In particular, it relates to emulsion compositions of the oil-in-water type, which provide suitable wipe-off or rinse-off make-up removing and skin cleansing compositions.

Emulsion type make-up removing compositions, containing oil, water and a surfactant, are known. However, conventional compositions provide a less than satisfactory performance, in terms of either their ability to remove make-up, or their aesthetic and sensory properties. In the case of oil based formulations, whilst these usually provide satisfactory make-up removal, they usually leave the user with a greasy afterfeel on the skin. With aqueous based products, there is a tendency for these to leave the skin feeling dry and tight after use.

The compositions of the current invention aim to remedy one or more of the deficiencies of the known compositions. In particular, it is an object of the invention to provide cleansing compositions which provide good cleansing and afterfeel properties. Preferably these will also confer to the skin a long term moisturising benefit.

Thus, according to the invention, there is provided a cleansing composition suitable for topical application to the human skin, comprising an oil-in-water emulsion system which comprises a hydroxy alkanoate derivative compound having the following general formula:

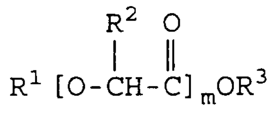

wherein

$R^1$ is H- or

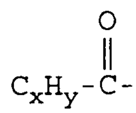

- $R^2$ is H- or $C_pH_q$-
- $R^3$ is H-, $C_xH_y$-, or a metallic, ammonium, or alkylammonium cation,
- p is an integer from 1 to 18
- q is an integer from 3 to 37
- x is an integer from 2 to 22,
- y is an integer from 3 to 43, and
- m is an integer from 1 to 3,

provided that when $R^1$ is H-, then $R^3$ is $C_xH_y$-, and when $R^1$ is $C_xH_yCO$- then $R^3$ is H-, or a metallic, ammonium or alkylammonium cation.

The invention also provides a method of removing make up from skin by applying a composition as above to the skin, and then wiping, washing or rinsing the emulsion from the skin.

The hydroxy alkanoate derivative above will generally function as an emulsifier which is part of all of an emulsifier system. However, it may be included as at least part of the oil of the emulsion - this applies particularly to lipophilic alkyl esters of hydroxy alkanoic acids, (in which compounds, $R^1$ above is H)

The hydroxy alkanoate derivatives employed in compositions according to the invention provide the cleansing composition with desirable properties, in the sense that they may not only act as an emulsifier within the composition, thereby stabilising the oil-in-water emulsion, but can also be beneficially metabolised in the skin.

During this process, naturally occurring enzymes in the skin cleave the hydroxy alkanoate derivative in the composition to generate lactic acid or glycolic acid (depending on whether $R^2$ is H or $CH_3$- in the above general formula), which may then permeate further through the skin. In particular, it is thought that the derivatives described penetrate the skin more readily than glycolic or lactic acids themselves, which are relatively hydrophilic.

Absorption of lactic acid, glycolic acid or other hydroxy alkanoic acid into the skin is desirable, because these compounds have a humectant action, they provide a moisturisation benefit, and they also improve the condition and feel of skin.

It has also been found to be a highly desirable aspect of the invention that the hydroxy alkanoate derivative used in compositions according to the invention has an hydrophilic/lipophilic balance (HLB) value of less than about 5.5. It is suspected that hydroxy alkanoate derivatives with this range of HLB values may more readily partition into the skin, and hence deliver a cosmetic benefit more deeply in the skin, because of their hydrophobicity.

In the formula given above $R^2$ is preferably H or $CH_3$, more preferably $CH_3$ so that the compound is a derivative of glycolic or lactic acid.

Lactic acid derivatives are readily available commercially, and preferred for this reason. However, homologues of the lactic derivatives are accessible and can also be used.

Acyl derivatives of 2-hydroxy alkanoate acids in which $R^2$ is $C_pH_q$ - and p is 2 or more can be made by reaction of an acyl halide with the 2-hydroxy alkanoic acid. Alkyl esters of 2-hydroxy alkanoic acids can be prepared by esterification of those acids.

If $R^2$ is $C_pH_q$ - and p is 2 or more, then p is preferably in the range 2 to 12, more preferably 2 to 6.

According to one preferred aspect of the invention, the hydroxy alkanoate derivative in the cleansing composition is an acyl lactylate; that is, in the general structure shown above, $R^3$ is H-, $R^2$ is $CH_3$ and $R^1$ is $C_xH_yCO$- . In a particularly preferred variant of acyl lactylates, in $R^1$ x is between 14 and 20, and is most preferably 17. In a further preferred variant of acyl lactylates, the $R^3$ species is a metallic cation which provides a relatively insoluble acyl lactylate species, such as a calcium or magnesium cation. A most preferred embodiment of the invention comprises calcium stearoyl lactylate, which has an HLB value of 5.1. Using such a relatively insoluble acyl lactylate may provide particularly beneficial properties to the composition.

Acyl lactylates are anionic surfactants, and are conveniently prepared by coupling lactic acid to long chain fatty acids via the alpha hydroxy group on the lactic acid. Such acyl lactylates surfactants are believed to readily break down in the skin to provide lactic acid, thereby providing the aforementioned beneficial absorption of lactic acid into the skin.

Another preferred category of hydroxy alkanoate derivatives for use in cleansing compositions according to the invention are alkyl lactates; that is $R^1$ is H- and $R^2$ is $CH_3$, and $R^3$ is $C_xH_y$. In a more preferred embodiment, x in $R^3$ is between 6 and 20, more preferably 12 and 18. In the most preferred embodiment, $R^3$ is 14.

Alkyl lactates are weak nonionic surfactants, in which the lactic acid moiety is linked to a long chain fatty alcohol through its carboxylate group. Alkyl lactates surfactants are essentially water insoluble, and therefore may provide a relatively poor foaming ability when compared to acyl lactylate surfactants, but may beneficially be used in compositions according to the invention, because of their ability to partition into the skin. This ability is often superior to that of the anionic acyl lactylate surfactants.

It is a characteristic of certain preferred embodiments of the invention that they may provide compositions which are relatively stable in use, thereby providing compositions which are commercially more attractive. By stable it is meant that the emulsion does not visibly breakdown to its constituent phases within one month of being prepared, in temperate conditions.

A preferred alkyl lactate for use in compositions according to the invention is myristyl lactate. This alkyl lactate has a very low HLB value, in the region 1-3.

The concentration of the hydroxy alkanoate derivative of of the general structure above in the composition is typically 0.5-10% by weight of the composition. However, the hydroxy alkanoate derivative of the general formula discussed is typically only one emulsifier which may be part of an emulsifier system used in compositions according to the invention. If used, such other emulsifiers may be used to make the total level of emulsifier system in compositions according to the invention to be between 0.5-10% by weight, more preferably 1-7% by weight.

Such other emulsifiers as may used in the emulsifier system according to the invention may include any emulsifier or combination of emulsifiers which has an average HLB value of greater than 6, more preferably greater than 9, and may be readily selected from any textbook on the subject.

The oil phase of compositions according to the invention may comprise mineral oils, hydrocarbons, silicone oils, triglyceride/ester oils, or mixtures thereof. Preferred oils are those which are particularly effective at solubilising the oil components of make up. Particularly preferred oils for use in compositions according to the invention are hydrocarbon oils, such as isoparaffins, polydecene and polybutene, mineral oils and esters which contain a saturated or unsaturated, straight or branched chain $C_{8-22}$ alkyl or alkenyl group, for example isopropylmyristate, hexyl laurate, methyl laurate, 2-ethylhexyl palmitate, 2-octadecyl myristate, isopropylpalmitate, and triglycerides such as glyceryl tricaprylate/caprate (eg ESTOL, ex. Unichema), glyceryl tri-isostearate (eg PRISORINE, ex. Unichema), and glyceryl tri- (2-ethylhexanoate) (eg MYRITOL GTEH, ex. Henkel).

Preferably, the composition comprises a hydrocarbon oil, or a hydrocarbon oil in combination with an ester oil as described above.

Particularly preferred oils for use in compositions according to the invention are isoparaffin and polydecene. These components are preferred components in the oil phase, since they are particularly effective in blending into and lifting a certain resilient make-up compositions which are typically resistant to removal, such as for example water-proof eye make-ups.

Preferably, the oil is present in the composition at a level of 3-60%, more preferably 10-40% by weight of the composition.

The water in compositions according to the invention may preferably comprise from 95-40%, more preferably 90-50% by weight of the composition.

The composition according to the invention may additionally comprise further adjuncts as are typically found in such cleansing compositions. These may typically include;

- thickeners, such as carbomers, xanthan gum, hectorite, and fumed silica,
- humectants, such as glycerol, propylene glycol, dipropylene glycol, sorbitol, and 2-pyrrolidone-5-carboxylate,
- perfumes,
- colourants,
- preservatives, such as salicylic acid, p-hydroxybenzoate esters, and 2-bromo-2-nitropropane-1,3-diol,
- antioxidants, such as butylated hydroxy toluene, tocopherol, and butylated hydroxy anisole,
- short-chain monohydric alcohols, such as ethanol and isopropanol,
- skin conditioners, such as polyquaternium 10 and PEG-7 glyceryl cocoate,
- germicides, such as triclosan and cetrimide, and
- plant extracts, such as aloe vera, cornflower, witch hazel, elderflower, and cucumber.

Products according to the invention may take any convenient product form, and as such may be in the form of lotions, creams, or may be readily combined in known ways with a suitable propellant to provide a product in mousse form.

## Examples

The invention will now be demonstrated with reference to the following example:

| Component | Composition (%w/w) 1 | 2 |
|---|---|---|
| Ethylflo 362 NF (1) | 20.0 | 20.0 |
| Permethyl 101A (2) | 10.0 | 10.0 |
| Cremophor RH40 (3) | 2.0 | - |
| Cremophor A6 (4) | - | 2.00 |
| Renex 30 (5) | 0.30 | 0.30 |
| Alfol 1618 (6) | 3.00 | 3.00 |
| Crodamol ML (7) | 3.00 | - |
| Crolactil CSL (8) | - | 5.00 |
| Nipasol M (9) | 0.10 | 0.10 |
| Nipagin M (10) | 0.20 | 0.20 |
| Carbopol 980 (11) | 0.30 | 0.30 |
| Triethanolamine | 0.40 | 1.36 |
| Water | 60.70 | 57.74 |
| | (pH 6.6) | (pH 5.9) |

(1) Polydecene dimer, ex. Ethyl Co.
(2) Isohexadecane, ex. Waren Empfanger
(3) PEG-40 hydrogenated castor oil, ex. BASF
(4) Ceteareth-6 and Stearyl alcohol, ex. BASF
(5) Trideceth-12, ex. ICI
(6) Cetearyl alcohol, ex. Condea
(7) Myristyl lactate, ex. Croda
(8) Calcium stearoyl lactylate ex. Croda
(9) Propyl paraben, ex. Nipa Labs.
(10) Methyl paraben, ex. Nipa Labs.
(11) Carbomer, ex. Goodrich

Compositions 1 and 2 were prepared by blending together the components of the composition as shown.

Preparation method

Compositions 1 and 2 were prepared according to the following general method.

Firstly the aqueous phase is prepared by blending the Nipagin and triethanolamine with an aliquot of water, and heating the mixture to 70-80°C. The oil phase is then prepared by adding the Ethylflo 362 NF, Permethyl 101A, Cremophor RH40, Renex 30, Alfol 1618, Nipasol, and the hydroxy alkanoate derivative to a container and heating to 70-80°C.

The oil phase is then blended into the aqueous phase using a Silverson mixer.

Into this is blended the polymeric thickener, which is pre-prepared by adding the Carbopol gradually, with stirring, into the remaining water, and subsequently heating this to 70-80°C.

The composition is finally adjusted to pH 6-6.6 using triethanolamine, where necessary.

Compositions 1 & 2 were evaluated for their make-up removal efficacy against a commercially available product, and were also tested for the deposition of lactic acid that occurred after use on human skin.

Make-up Removal Efficacy

Compositions 1 and 2 were assessed for their make-up removal efficacy, against the commercially available product Oil of Ulay Water Rinsable Cold Cream. This product contains water, mineral oil, polyalphaolefin, glycerin, isododecane, sorbitan stearate, cyclomethicone, caprylic/capric triglyceride, hexylene glycol, PEG-8 laurate, stearyl alcohol, Ceteareth-20, lauryl glycoside, acrylic acid copolymer, castor oil, cetyl alcohol, dioctyl sodium sulphosuccinate, methylchloroisothiazolinone and methylisothioazolinone, PEG-10 soya sterol, triethanolamine, and fragrance.

Test Protocol

Approximately 0.015g of Shiseido Waterproof Eyeliner is then applied to three 3cmx2cm designated patches on a tester's forearm. Prior to application, the colour of the skin is measured using a Minolta Chroma Meter CR-100, to give a set of baseline readings (A). The 3-D colour co-ordinate system L*a*b was used, and each recorded result was the average of 6 individual measurements. A panel of 9 people were used. After application and drying, a second colour measurement was taken (B)

To this is applied 0.2 g of the cleansing composition being tested, the composition being gently rubbed into the test area with either 40 or 100 light circular rubbing motions. The composition is subsequently removed either by wiping off with a tissue in two motions, forward and back, or rinsing with water at flow rate of approx. 6 l/min, and a temperature of approximately 35°C. All patches are dried, and final colour measurements are taken (reading C).

The percentage removal is calculated as (B-C)/(B-A)x 100 , where B-A is the difference in L, a, and b readings, and so on for B-C.

Results

| | % make-up removal | |
|---|---|---|
| **Composition** | **40 rubs/wipe off** | **100 rubs/rinse off** |
| 1 | 46.9% | 43.4% |
| 2 | 58.0% | 65.6% |
| Oil of Ulay Water Rinsable Cold Cream | 40.0% | 37.1% |

As can be seen from the above results, compositions 1 and 2 provided superior make-up removal efficacy to the commercially available formulations under both test protocols, and may also deliver to the skin a longer term skin benefit, due to absorption by the skin of the hydroxy alkanoate derivative.

Example 2

The following composition provides a cleansing milk composition.

| Component | %(w/w) |
|---|---|
| Cetearyl alcohol (Alfol1618, ex. Condea) | 2.0 |
| Trioctanoin (Estol3609, ex. Unichema) | 5.0 |
| Polydecene (Ethylflo 362NF, ex. Ethyl) | 5.0 |
| Calcium stearoyl lactylate (Crolactil CSL, ex Croda) | 1.0 |
| Ceteth 21 (Brij 721, ex. ICI) | 3.0 |
| Ceteth 2 (Brij 72, ex. ICI) | 1.0 |
| Propyl Paraben (Nipasol, ex. Nipa) | 0.2 |
| Glycerine (ex. Unichema) | 2.0 |
| Methyl Paraben (Nipagin M, ex.Nipa) | 0.2 |
| Xanthan gum (Kelzan, ex. Kelco) | 0.1 |
| Perfume | q.v |
| Water | to 100 |

Example 3

The following composition provides a cleansing lotion according to the invention.

| Component | %(w/w) |
|---|---|
| $C_{11}$-$C_{13}$ isoparaffin (Isopar L, ex. Exxon) | 5.0 |
| Caprylic/capric triglyceride (Miglyol 810, ex.Huls) | 15.0 |
| Myristyl lactate (Crodamol ML, ex. Croda) | 2.0 |
| Cetearyl alcohol (Alfol 1618, ex. Condea) | 3.0 |
| Ceteth 21 (Brij 721, ex. ICI) | 1.0 |
| Propyl paraben (Nipasol M, ex. Nipa) | 0.1 |
| Propylene glycol (ex. BASF) | 3.0 |
| Triethanolamine (ex. BASF) | 0.2 |
| Carbomer 934 (Carbopol 934, ex. Goodrich) | 0.2 |
| Methyl paraben (Nipagin M, ex. Nipa) | 0.2 |
| DMDM hydantoin (Glydant, ex. Lonza) | 0.2 |
| Perfume | q.v |
| Water | to 100 |

Example 4

The following composition provides an aerosol cleansing mousse composition according to the invention.

| Component | %(w/w) |
|---|---|
| Isopropyl myristate (Estol 1514, ex. Unichema) | 25.0 |
| PEG-8 laurate (Clihrol 4ML, ex. Croda) | 6.0 |
| Myristyl lactate (Crodamol ML, ex. Croda) | 2.0 |
| Glycerine (ex. Unichema) | 2.0 |
| Butane (Cap 30, ex. Calor) | 5.0 |
| Water | to 100 |

## Claims

1. A skin cleansing composition for topical application to the human skin to remove make up therefrom comprising an oil-in-water emulsion containing a hydroxy alkanoate derivative compound of the following general formula:

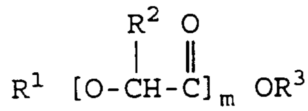

wherein

$R^1$ is H- or

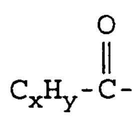

$R^2$ is H- or $C_pH_q$-
$R^3$ is H-, $C_xH_y$-, or a metallic, ammonium, or alkylammonium cation,
p is an integer from 1 to 18,
q is an integer from 3 to 37,
x is an integer from 2 to 22,
y is an integer from 3 to 43, and
m is an integer from 1 to 3,

provided that when $R^1$ is H-, then $R^3$ is $C_xH_y$-, and when $R^1$ is $C_xH_yCO$-, then $R^3$ is H-, or a metallic, ammonium or alkylammonium cation.

2. A cleansing composition according to claim 1 wherein $R^2$ is H or $CH_3$.

3. A cleansing composition according to claim 1 or claim 2, wherein the hydroxyalkanoate derivative has an HLB value of less than 5.5.

4. A cleansing composition according to any one of the preceding claims, wherein the oil phase comprises a mineral oil, a hydrocarbon oil, or an ester which contains a saturated or unsaturated straight or branched $C_8$-$C_{22}$ alkyl or alkenyl chain, or mixture thereof.

5. A cleansing composition according to any one of the preceding claims wherein the amount of oil in the oil phase of the emulsion is 3 to 60% by weight of the composition and the amount of the hydroxy alkanoate derivative is 0.5 to 7% by weight of the composition.

6. A method of removing cosmetics from human skin, comprising:

(i) applying to skin with make up thereon a skin cleansing composition which his an oil-in-water emulsion containing a hydroxy alkanoate derivative compound of the following general formula:

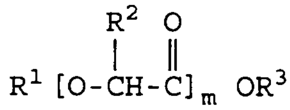

wherein

$R^1$ is H- or

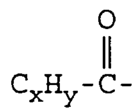

$R^2$ is H- or $CH_3$
$R^3$ is H-, $C_xH_y$-, or a metallic, ammonium, or alkylammonium cation,
p is an integer from 1 to 18,
q is an integer from 3 to 37,
x is an integer from 2 to 22,
y is an integer from 3 to 43, and
m is an integer from 1 to 3,

provided that when $R^1$ is H-, then $R^3$ is $C_xH_y$-, and when $R^1$ is $C_xH_yCO$-, then $R^3$ is H-, or a metallic, ammonium or alkylammonium cation, and

(ii) wiping, washing or rinsing said emulsion from the skin.

7. A method according to claim 6 wherein $R^2$ is H or $CH_3$.

8. A method according to claim 6 or claim 7, wherein the hydroxyalkanoate derivative has an HLB value of less than 5.5.

9. A method according to any one of claims 6 to 8, wherein the oil phase comprises a mineral oil, a hydrocarbon oil, or esters which contain a saturated or unsaturated straight or branched chain $C_8$-$C_{22}$ alkyl or alkenyl group, or mixtures thereof.

10. A method according to any one of claims 6 to 9 wherein the amount of the oil is 3 to 60% by weight of the emulsion and the amount of the hydroxyalkanoate derivative is 0.5 to 7% by weight of the emulsion.

11. Use of a hydroxyalkanoate derivative as defined in any one of claims 1 to 3 as an emulsifier in a composition for removing make up from human skin.

**Patentansprüche**

1. Eine Haut-Reinigungszusammensetzung für die örtliche Anwendung auf die menschliche Haut zur Entfernung des Make-up von derselben, umfassend eine Öl-in-Wasser-Emulsion, enthaltend eine Hydroxyalkanoatderivat-Verbindung der nachfolgenden allgemeinen Formel:

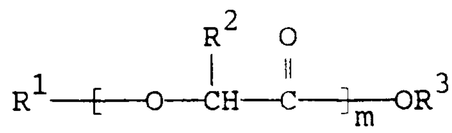

worin

$R^1$ H- oder

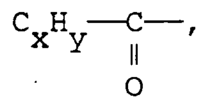

$R^2$ H- oder $C_pH_q$-,
$R^3$ H-, $C_xH_y$-, oder ein metallisches, Ammonium- oder Alkylammonium-Kation,
p eine ganze Zahl von 1 bis 18,
q eine ganze Zahl von 3 bis 37,
x eine ganze Zahl von 2 bis 20,
y eine ganze Zahl von 1 bis 3,
bedeuten,

vorausgesetzt, daß, falls $R^1$ H- bedeutet, dann $R^3$ $C_xH_y$- ist, und, falls $R^1$ $C_xH_yCO$- ist, dann $R^3$ H-, oder ein metallisches, Ammonium- oder Alkylammonium-Kation ist.

2. Eine Reinigungszusammensetzung nach Anspruch 1, worin $R^2$ H oder $CH_3$ ist.

3. Eine Reinigungszusammensetzung nach Anspruch 1 oder Anspruch 2, worin das Hydroxyalkanoat-Derivat einen HLB-Wert von weniger als 5,5 aufweist.

4. Eine Reinigungszusammensetzung nach einem der vorstehenden Ansprüche, worin die Ölphase ein Mineralöl, ein Kohlenwasserstofföl oder einen Ester umfaßt, der eine gesättigte oder ungesättigte, geradkettige oder verzweigtkettige $C_{8-22}$-Alkyl- oder -Alkenylgruppe, oder Mischungen derselben, enthält.

5. Eine Reinigungszusammensetzung nach einem der vorstehenden Ansprüche, worin die Menge des Öls in der Ölphase der Emulsion 3 bis 60 Gewichtsprozent der Zusammensetzung und die Menge des Hydroxyalkanoat-Derivats 0,5 bis 7 Gewichtsprozent der Zusammensetzung beträgt.

6. Ein Verfahren zur Entfernung der Kosmetika von der menschlichen Haut, umfassend:

(i) Aufbringen einer Haut-Reinigungszusammensetzung auf die mit Make-up versehene Haut, welche eine Öl-in-Wasser-Emulsion ist, enthaltend eine Hydroxyalkanoatderivat-Verbindung der nachfolgenden allgemeinen Formel:

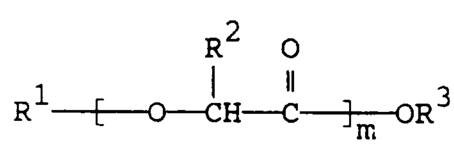

worin

$R^1$ H- oder

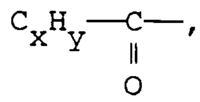

$R^2$ H- oder $CH_3$,
$R^3$ H-, $C_xH_y$-, oder ein metallisches, Ammonium- oder Alkylammonium-Kation,
p eine ganze Zahl von 1 bis 18,
q eine ganze Zahl von 3 bis 37,
x eine ganze Zahl von 2 bis 20,
y eine ganze Zahl von 1 bis 3,
bedeuten,

vorausgesetzt, daß, falls $R^1$ H- bedeutet, dann $R^3$ $C_xH_y$- ist, und, falls $R^1$ $C_xH_yCO$- ist, dann $R^3$ H-, oder ein metallisches, Ammonium- oder Alkylammonium-Kation ist, und
(ii) Wischen, Waschen oder Spülen der erwähnten Emulsion von der Haut.

7. Ein Verfahren nach Anspruch 6, worin $R^2$ H oder $CH_3$ ist.

8. Ein Verfahren nach Anspruch 6 oder Anspruch 7, worin das Hydroxyalkanoat-Derivat einen HLB-Wert von weniger als 5,5 aufweist.

9. Ein Verfahren nach einem der Ansprüche 6 bis 8, worin die Ölphase ein Mineralöl, ein Kohlenwasserstofföl oder Ester umfaßt, welche eine gesättigte oder ungesättigte, geradkettige oder verzweigtkettige $C_{8-22}$-Alkyl- oder -Alkenylgruppe, oder Mischungen derselben, enthalten.

10. Ein Verfahren nach einem der Ansprüche 6 bis 9, worin die Menge des Öls 3 bis 60 Gewichtsprozent der Emulsion, und die Menge des Hydroxyalkanoat-Derivats 0,5 bis 7 Gewichtsprozent der Emulsion beträgt.

11. Die Verwendung eines Hydroxyalkanoat-Derivats, wie in einem der Ansprüche 1 bis 3 definiert, als Emulgiermittel in einer Zusammensetzung zur Entfernung des Make-up von der menschlichen Haut.

## Revendications

1. Composition de nettoyage de la peau pour application locale à la peau humaine pour enlever le maquillage comprenant une émulsion huile-dans-eau contenant un composé dérivé d'hydroxyalcanoate de formule générale suivante :

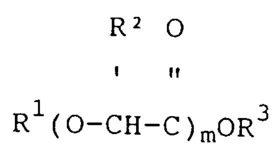

dans laquelle

$R^1$ est H- ou

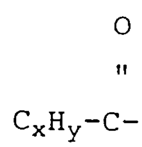

$R^2$ est H- ou $C_pH_q$-
$R^3$ est H-, $C_xH_y$- ou un cation métallique, ammonium ou alkylammonium,
p est un nombre entier de 1 à 18,
q est un nombre entier de 3 à 37,
x est un nombre entier de 2 à 22,
y est un nombre entier de 3 à 43, et
m est un nombre entier de 1 à 3,

à la condition que quand $R^1$ est H-, alors $R^3$ est $C_xH_y$- et quand $R^1$ est $C_xH_yCO$-, alors $R^3$ est H- ou un cation métallique, ammonium ou alkylammonium.

2. Composition de nettoyage selon la revendication 1, dans laquelle $R^2$ est H ou $CH_3$.

3. Composition de nettoyage selon la revendication 1 ou 2, dans laquelle le dérivé d'hydroxyalcanoate a un I.A de moins de 5,5.

4. Composition de nettoyage selon l'une quelconque des revendications précédentes, dans laquelle la phase huileuse comprend une huile minérale, une huile hydrocarbonée ou un ester qui contient une chaîne alkyle ou alcényle en $C_{8-22}$ droite ou ramifiée, saturée ou insaturée, ou un mélange de ceux-ci.

5. Composition de nettoyage selon l'une quelconque des revendications précédentes, dans laquelle la quantité d'huile dans la phase huileuse de l'émulsion est 3 à 60% en poids de la composition et la quantité de dérivé d'hydroxyalcanoate est 0,5 à 7% en poids de la composition.

6. Procédé pour enlever les cosmétiques de la peau humaine, consistant :

(i) à appliquer à la peau comportant du maquillage une composition de nettoyage de la peau qui est une émulsion huile-dans-eau contenant un composé dérivé d'hydroxyalcanoate de la formule générale suivante :

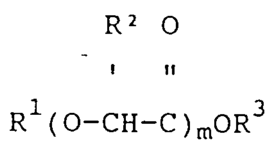

dans laquelle

$R^1$ est H- ou

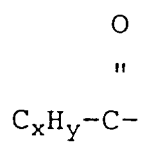

$R^2$ est H- ou $C_pH_q$-
$R^3$ est H-, $C_xH_y$- ou un cation métallique, ammonium ou alkylammonium,
p est un nombre entier de 1 à 18,
q est un nombre entier de 3 à 37,
x est un nombre entier de 2 à 22,
y est un nombre entier de 3 à 43, et
m est un nombre entier de 1 à 3,

à la condition que quand $R^1$ est H-, alors $R^3$ est $C_xH_y$- et quand $R^1$ est $C_xH_yCO$-, alors $R^3$ est H- ou un cation

métallique, ammonium ou alkylammonium, et

(ii) à essuyer, laver ou rincer ladite émulsion à partir de la peau.

**7.** Procédé selon la revendication 6, dans lequel $R^2$ est H ou $CH_3$.

**8.** Procédé selon la revendication 6 ou 7, dans lequel le dérivé d'hydroxyalcanoate a un I.A de moins de 5,5.

**9.** Procédé selon l'une quelconque des revendications 6 à 8, dans lequel la phase huileuse comprend une huile minérale, une huile hydrocarbonée ou des esters qui contiennent un groupe alkyle ou alcényle en $C_{8-22}$ à chaîne droite ou ramifiée, saturé ou insaturé, ou un mélange de ceux-ci.

**10.** Procédé selon l'une quelconque des revendications 6 à 9, dans laquelle la quantité d'huile dans la phase huileuse de l'émulsion est 3 à 60% en poids de l'émulsion et la quantité de dérivé d'hydroxyalcanoate est 0,5 à 7% en poids de l'émulsion.

**11.** Utilisation d'un dérivé d'hydroxyalcanoate comme défini dans l'une quelconque des revendications 1 à 3 comme émulsifiant dans une composition pour enlever le maquillage de la peau humaine.